(19) **Europäisches Patentamt / European Patent Office / Office européen des brevets**

(11) **EP 1 496 875 B1**

(12) **EUROPÄISCHE PATENTSCHRIFT**

(45) Veröffentlichungstag und Bekanntmachung des Hinweises auf die Patenterteilung:
**14.05.2008 Patentblatt 2008/20**

(51) Int Cl.:
*A61K 9/70* (2006.01)   *A61K 35/20* (2006.01)
*A61P 17/00* (2006.01)

(21) Anmeldenummer: **03718537.8**

(22) Anmeldetag: **23.04.2003**

(86) Internationale Anmeldenummer:
**PCT/AT2003/000116**

(87) Internationale Veröffentlichungsnummer:
**WO 2003/090728 (06.11.2003 Gazette 2003/45)**

(54) **VERWENDUNG EINES AUF EINER BIOLOGISCH INERTEN, HOCHDISPERSEN MATRIX GETROCKNETEN STUTENMILCHKONZENTRATS**

USE OF A MARE'S MILK CONCENTRATE DRIED ON A HIGHLY-DISPERSED, BIOLOGICALLY INERT MATRIX

UTILISATION D'UN CONCENTRE DE LAIT DE JUMENT SECHE SUR UNE MATRICE BIOLOGIQUEMENT INERTE, FORTEMENT DISPERSEE

(84) Benannte Vertragsstaaten:
**AT BE BG CH CY CZ DE DK EE ES FI FR GB GR HU IE IT LI LU MC NL PT RO SE SI SK TR**
Benannte Erstreckungsstaaten:
**LT LV MK**

(30) Priorität: **25.04.2002 AT 6402002**

(43) Veröffentlichungstag der Anmeldung:
**19.01.2005 Patentblatt 2005/03**

(73) Patentinhaber: **SOBA Biotec GmbH**
**53604 Bad Honnef (DE)**

(72) Erfinder:
• **KUKLINSKI, Bodo**
**18055 Rostock (DE)**
• **SCHIEFER, Raimund**
**A-5580 Tamsweg (AT)**
• **MARKOLIN, Gertrude**
**A-5571 Mariapfarr (AT)**
• **KÖSSLER, Peter**
**A-5571 Mariapfarr (AT)**
• **FUCHS, Norbert**
**A-5571 Mariapfarr (AT)**

(74) Vertreter: **Sonn & Partner Patentanwälte**
**Riemergasse 14**
**1010 Wien (AT)**

(56) Entgegenhaltungen:
**US-A- 4 559 222**

• **DATABASE WPI Section Ch, Week 199322 Derwent Publications Ltd., London, GB; Class B04, AN 1993-180727 XP002247615 & SU 1 740 002 A (AS MED LOCAL FEEDING PROBL INST), 15. Juni 1992 (1992-06-15)**
• **DATABASE WPI Section Ch, Week 200121 Derwent Publications Ltd., London, GB; Class D21, AN 2001-203417 XP002247616 & CN 1 275 374 A (WU J), 6. Dezember 2000 (2000-12-06)**
• **CAMPIGLI V ET AL: "Silicon dioxide as support for amorphous and metastable crystalline forms of indomethacin. Correlation between drug thermodynamic activity and percutaneous absorption in vitro" FARMACO, EDIZIONE PRATICA 1988 ITALY, Bd. 43, Nr. 2, 1988, Seiten 57-70, XP009013847 ISSN: 0014-827X**
• **RAETZ K H ET AL: "Effects of some topically applied drugs on the skin surface lipids" DERMATOLOGISCHE MONATSSCHRIFT 1975, Bd. 161, Nr. 11, 1975, Seiten 948-951, XP009013852**
• **MUELLER-GOYMANN C C: "NEUERE HILFSSTOFFE" DEUTSCHE APOTHEKER ZEITUNG, DEUTSCHER APOTHEKER ZEITUNG, STUTTGART, DE, Bd. 132, Nr. 43, 22. Oktober 1992 (1992-10-22), Seiten 2306-2311, XP000306320 ISSN: 0011-9857**

## Beschreibung

Die vorliegende Erfindung betrifft die Verwendung eines auf einer biologisch inerten, hochdispersen Matrix getrockneten Stutenmilchkonzentrats zur Herstellung eines Präparats zur oralen Aufnahme zur Behandlung von Hauterkrankungen, insbesondere trockenen Hauterkrankungen.

Neurodermitis (syn. AtopiscHe Dermatitis; Atopisches Ekzem; Endogenes Ekzem) zählt zu den chronischen bzw. chronisch-rezidivierenden Hauterkrankungen. Klinisch äußert sich die Neurodermitis im frühen Kindesalter mit Juckreiz, Rötung, Schuppung, Nässen und Krustenbildung vor allem an den Wangen (Milchschorf), an den Ohren oder in verschiedenen Faltenregionen. Diese milden Formen der Neurodermitis werden häufig nicht als Neurodermitis diagnostiziert und daher auch nicht adäquat therapiert. Etwa ab dem zweiten Lebensjahr entspricht das klinische Bild der Neurodermitis jenem von Erwachsenen, wobei in diesem Stadium vor allem das Beugeekzem (Eczema flexurarum) imponiert. Im späteren Schulalter und in der Pubertät zeigt sich eine dritte Verlaufsform als "Neurodermitis disseminata", wobei der ganze Körper von Ekzemherden (Gesicht, Stamm, Extremitäten, Gelenksbeugen) betroffen sein kann.

Die Ätiopathogenese gilt als weitgehend ungeklärt, wobei folgende Faktoren als Auslöser und/oder Promotoren des Krankheitsbildes diskutiert werden: eine genetische Prädisposition (autosomal dominanter Erbgang), neurovegetative Regulationsstörungen der Vasomotorik, psychische Faktoren (berufliche und/oder familiäre Veränderungen, Überbelastungen, Partner- oder Familienprobleme), exogene Faktoren (Allergene, Klima), intestinale Candidose, immunologische Faktoren (IGE-vermittelte Überempfindlichkeitsreaktion vom Soforttyp, resp. Typ I der Allergie) sowie enzymatische Defekte (eingeschränkte Aktivität des Enzyms delta-6-Desaturase).

Der multifaktoriellen Genese der Neurodermitis entsprechend variantenreich ist das therapeutische Angebot: symptomatische Behandlung mit Antihistaminen (innerlich und äußerlich), Glukokortikoiden (innerlich und äußerlich), Benzodiazepinen (da der quälende Juckreiz vor allem Nachts auftritt), Öl- und Teerbäder, Klimatherapie im Gebirgs- oder Meeresklima, harnstoffhaltige Externa, antimykotische Präparate (innerlich und äußerlich), UV-Therapie sowie Linolensäure-haltige Pflanzenöle zur innerlichen Anwendung.

Europaweit dürften sechs bis acht Millionen Patienten an Neurodermitis leiden, jährlich werden etwa dreihunderttausend Neuerkrankungen gemeldet. Während bei den Erwachsenen lediglich 0,7 % der Bevölkerung betroffen sind, leiden 10 bis 15 % der europäischen Kinder an atopischen Erkrankungen, sodass die Neurodermitis vor allem ein pädiatrisches Problem darstellt.

Auf physiologischer bzw. biochemischer Ebene wird als Ursache eine Einschränkung der Aktivität des Enzyms delta-6-Desaturase diskutiert. Dieses Enzym katalysiert die Umwandlung der essentiellen omega-6-Fettsäure "Linolsäure" (C18:2) in gamma-Linolensäure (C18:3), welche ihrerseits in einem nachfolgenden Schritt zu Dihomo-gamma-Linolensäure (C20:3) elongiert wird und das physiologische Ausgangsprodukt für Serie-Eins-Prostaglandine ($PGE_1$) darstellt. Serie-Eins-Prostaglandine wirken entzündungshemmend und gefäßerweiternd und sind bei Atopikern im Vergleich zu Gesunden erniedrigt. Nachdem Atopiker einerseits erhöhte Linolsäurekonzentrationen und andererseits um mehr als die Hälfte reduzierte gamma-Linolensäurespiegel im Plasma aufweisen, gilt die "delta-6-Desaturase-Hypothese" als weitgehend gesichert. Die katalytischen Funktionen sowie die Aktivität der delta-6-Desaturasen sind Eisen (hämin- und nicht-hämingebunden)-, Niacin (NADH bzw. NADPH)- sowie Riboflavin ($FADH_2$)-abhängig. Aus der oben erwähnten Dihomo-gamma-Linolensäure entsteht neben PGE, auch Arachidonsäure (C20:4), welche ihrerseits biochemischer Precursor von Prostacyclinen, Thromboxanen und Leukotrienen ist. Inwieweit Immun-Modulatoren das pathologische Geschehen der Neurodermitis beeinflussen, ist derzeit noch Gegenstand zahlreicher Untersuchungen.

Andere Untersuchungen lassen vermuten, dass der Neurodermitis eine pathophysiologische Reifungsstörung der T-Lymphozyten im Thymus und/oder in der Epidermis zugrundeliegt.

Aufgrund dieser Reifungsstörung kommt es zu einer unkontrollierten kutanen T-Zell-Infiltration. Als gesichert gilt jedenfalls, dass die essentiellen Fettsäuren (die omega-3-Fettsäure "alpha-Linolensäure" sowie die omega-6-Fettsäure "Linolsäure") und die daraus gebildeten Eicosanoide gleichermaßen die Integrität der Epidermis als auch die Funktionstüchtigkeit des Immunsystems wesentlich beeinflussen. Dabei werden die immunregulatorischen Effekte insbesondere der essentiellen omega-6-Fettsäuren durch Serie-Eins-Prostaglandine ($PGE_1$) vermittelt und moduliert.

Eine weitere, zusätzliche Komplikation der Neurodermitis ist das Auftreten bakterieller oder viraler Sekundärinfektionen, provoziert durch ständiges Kratzen an den betroffenen juckenden Hautstellen.

Psoriasis (Schuppenflechte) ist eine der häufigsten Hautkrankheiten von Erwachsenen. Ein bis zwei Prozent aller Europäer sind von dieser nicht ansteckenden, schubweise auftretenden Hautkrankheit betroffen. Sie wird wahrscheinlich durch ein immunpathogenetisches Geschehen in der Haut, die zu einer Entzündung und einer massiven Hyperproliferation von Keratinozyten und damit überschneller Bildung der Oberhaut verursacht. Ursache sind vermutlich genetische Faktoren.

Entzündliche Prozesse, Verletzungen und psychosomatische Störungen begünstigen den Ausbruch der Krankheit. Die Therapie der Psoriasis wird durch zwei wesentliche Faktoren bestimmt. Zum einen handelt es sich um eine chronisch rezidivierende Erkrankung, die über einen sehr langen Zeitraum eine Behandlung erfordern kann, zum anderen müssen Individualfaktoren wie internistische Begleiterkrankungen, ebenso wie klinische Formen der Psoriasis und Vorbehandlung beachtet werden. Die Therapie erfolgt als Lokaltherapie und/oder systemische Therapie sowie in Form einer Phototherapie, die mit anderen Therapieformen kombiniert werden kann. Es kommt dabei zu einer Linderung, jedoch nicht zur Heilung der Erkrankung.

Phänotypische Ausprägung und verlauf sind variabel. Leichte Verlaufsformen zeigen einzelne Herde an Prädilektionsstellen an, die jahrelang persistieren können oder mit unterschiedlich langen Zeiten völliger Erscheinungsfreiheit wechseln. Schwere Formen sind durch großflächiges Auftreten psoriatischer Effloreszenzen gekennzeichnet, spontane Rückbildung der Läsionen ist selten. Schwerste Ausprägung ist die Erythrodermie sowie die generalisierte pustulöse Psoriasis. Beide Formen zeigen Allgemeinsymptome. Vom klinischen Erscheinungsbild ist die Psoriasis vulgaris mit 90 % die am häufigsten auftretende Form der Schuppenflechte. Scharf begrenzte erythematöse Papeln und Plaques mit einer groblamellären, silbrig glänzenden Schuppung prägen die typische Morphologie. Die Prädilektionsstellen sind die Streckseiten der Ellbogen und Knie, periumbilikal und sakral, jedoch finden sich oft auch ausgedehnte Herde an der Kopfhaut. Die Psoriasis guttata (eruptive, kleinfleckige Psoriasis) entwickelt sich vor allem bei jüngeren Patienten, in der Folge von Streptokokken-Infektionen der oberen Atemwege, als Erstmanifestation. Die generalisierte Psoriasis pustulosa (von Zumbusch) ist die schwerste Verlaufsform der Psoriasis, bei der schubweise, von Fieberattacken begleitet, das gesamte Integument sich pustulös umwandelt. Zu den lokalisierten Formen zählt die Pustulosis palmo-plantaris an Handflächen und Fußsohlen und die sehr seltene Akrodermatitis continua suppurativa. Bei 10 bis 30 % der Psoriatiker findet man eine Psoriasis-Arthritis. Diese ist meist mit psoriatischen Veränderungen der Finger- und Zehennägel vergesellschaftet und kann dem Auftreten der Hautveränderung vorausgehen.

Berichte aus der Ernährungsheilkunde beschreiben die erfolgreiche Anwendung von nativer Stutenmilch unter anderem auch bei Neurodermitis sowie Psoriasis. Im Vergleich zu Kuhmilch zeigt Stutenmilch eine der humanen Milch sehr ähnliche Zusammensetzung und weist einen höheren Anteil an essentiellen, hoch ungesättigten Fettsäuren auf und für den Hautstoffwechsel wichtige Phospholipide, obwohl der absolute Fettgehalt von Stutenmilch geringer ist als jener von Kuhmilch. Stutenmilch enthält daneben noch überdurchschnittlich hohe Anteile an natürlichen antioxidativen Nährstoffen wie E-Vitamine, Vitamin C und Vitamin B12.

Beispielsweise beschreibt Alexander Bühlbäcker in "Zur Verwendbarkeit von Stutenmilch, Kumyß und Eselmilch als Diätetika und Heilmittel unter besonderer Berücksichtigung der Bedürfnisse des Säuglings und des Frühgeborenen" (Verlag Dr. Markus Hänsel-Hohenhausen (1996), Seiten 367-376) die Verwendung von nativer Stutenmilch als Nahrungsmittelzusatz bei der Behandlung von Neurodermitis. Aus diesen Falldarstellungen geht hervor, dass bei der diätetischen Behandlung von Neurodermitis mit nativer Stutenmilch eine Mindestbehandlungsdauer von zehn Monaten notwendig ist und dass Stutenmilch alleine, d.h. ohne zusätzliche therapeutisch und diätetische Maßnahmen, nicht wirksam ist. Weiters besteht bei nativer Stutenmilch das Problem der Lagerung, da diese bei Raumtemperatur nicht lagerstabil ist. Native Stutenmilch ist bei Zimmertemperatur nur wenige Tage, gekühlt nur etwa eine Woche und tiefgefroren maximal ein halbes Jahr haltbar.

Um das Problem der geringen Lagerstabilität zu umgehen wurden getrocknete Stutenmilchprodukte, insbesondere in Form von Pulver oder Kapseln, hergestellt. Die Trocknung geschieht dabei beispielsweise durch Gefriertrocknung, die jedoch unwirtschaftlich ist, durch Sprühtrocknung, wobei hochwertige Proteine zerstört werden, und durch Evaporation, wobei als Rückstand eine amorphe Masse mit begrenzter Lagerfähigkeit zurückbleibt.

Es besteht daher der Bedarf nach einem zu nativer Stutenmilch alternativen Präparat zur Behandlung von (trockenen) Hauterkrankungen, insbesondere Neurodermitis und Psoriasis, welches ohne weitere zusätzliche therapeutische und diätetische Maßnahmen wirksam sein soll, wobei die Heilung bzw. Besserung bereits nach kurzer Behandlungsdauer einsetzen soll und wobei das zu verabreichende Produkt auch bei Raumtemperatur längerfristig lagerstabil sein soll. Weiters ist es wichtig, dass ein solches haltbares Produkt eine hohe biologischer Wertigkeit aufweist.

Die Aufgabe wird gelöst durch die Verwendung eines auf einer biologisch inerten, hochdispersen Matrix getrockneten Stutenmilchkonzentrats zur Herstellung eines Präparats zur oralen Aufnahme zur Behandlung von Hauterkrankungen, insbesondere trockenen Hauterkrankungen.

Unter "trockenen Hauterkrankungen" werden dabei beispielsweise trockene Altershaut, Psoriasis, Neurodermitis u.Ä. verstanden.

Es hat sich überraschenderweise gezeigt, dass ein auf einer biologisch inerten hochdispersen Matrix getrocknetes (haltbares) Stutenmilchkonzentrat sich ausgesprochen gut zur Behandlung von Hauterkrankungen eignet und im Vergleich zur nativen Stutenmilch eine hohe Lagerstabilität auch bei Raumtemperatur aufweist.

Das durch das erfindungsgemäße Verfahren gewonnene Stutenmilch-Trockenkonzentrat weist eine Haltbarkeit von 24 bis 36 Monaten auf. Dieser technologische Vorgang macht es somit, möglich, Stutenmilch mit anderen funktionellen Nährstoffen (im speziellen Falle mit hautwirksamen Vitaminen, Mineralstoffen, Spurenelementen, hochungesättigten Fettsäuren) zu kombinieren.

Die Herstellung solcher Stutenmilchkonzentrate ist wie oben beschrieben bereits bekannt, da technologische Verfahren entwickelt wurden, um die Haltbarkeit von Stutenmilch bei Raumtemperatur von wenigen Tagen auf wenigstens zwei Jahre zu verlängern, ohne bei dem Trocknungsverfahren die temperatur- und sauerstoffsensiblen Inhaltsstoffe von Stutenmilch zu zerstören.

Zu diesem Zwecke wurden beispielsweise verfahren der Vakuum-Evaporation beschrieben, um so bei einer Temperatur von unter 40°C und unter Sauerstoff-Ausschluss das in der Milch enthaltene Wasser zu entfernen und so die Stutenmilch zu trocknen und aufzukonzentrieren. Aufgrund des Gehaltes an niedermolekularen Oligosacchariden, Oligopeptiden sowie hochwertigen Ölen stellt sich das Stutenmilch-Konzentrat als viskos-amorphe Masse dar, die in dieser Form galenisch nur schwer verarbeitbar ist. Um diesen technologischen Nachteil auszugleichen, wurde beispielsweise in der AT 393 961 offenbart, dass Stutenmilch vor der Vakuum-Destillation mit inertem, hochdispersem Siliciumdioxid (Kieselerde) als Trägermatrix versetzt werden soll, sodass sich nach der Vakuum-Destillation ein kristallines, pulveriges Trockenkonzentrat ergibt.

Diese Stutenmilch-Trockenkonzentrate mit hochdisperser Matrix wurden somit entwickelt, um das Herstellungsverfahren zu vereinfachen unter Beibehaltung der hochwertigen Inhaltsstoffe, sowie auch um die Stutenmilch über längere Zeit hinweg lagern zu können, ohne dass es zu Qualitätseinbußen kommt. Durch das Siliciumdioxid erhält das Produkt weiters eine verbesserte Rieselfähigkeit. Als Verwendung dieses Trockenmilchkonzentrats wird der Einsatz als Immunstimulator beschrieben. Die Verwendung dieses speziellen Konzentrats zur Behandlungen von Hauterkrankungen wurde jedoch bisher weder beschrieben noch nahegelegt.

Es hat sich nun erstmals überraschenderweise herausgestellt, dass sich dieses spezielle Stutenmilch-Trockenkonzentrat besonders gut zur Behandlung von Hauterkrankungen eignet. Beispielsweise im Vergleich zur gefriergetrockneten Stutenmilch weist das erfindungsgemäße Stutenmilchkonzentrat Vorteile in der Behandlung von Hauterkrankungen auf, da durch die schonende Trocknung, die durch die gleichmäßige Verteilung der Stutenmilch auf der hochdispersen Matrix ermöglicht wird, die biologische Wertigkeit der nativen Stutenmilch voll erhalten bleibt.

Unter dem Begriff "hochdisperse Matrix" kann erfindungsgemäß eine Matrix mit großer Oberfläche von zumindest 50 m$^2$/g verstanden werden. Dabei ist es wichtig, dass die Matrix biologisch inert ist, sodass die Stutenmilch chemisch nicht verändert wird und so an biologischer Wertigkeit verliert. Dadurch, dass die Stutenmilch auf der hochdispersen Matrix getrocknet wird, wird erreicht, dass die Stutenmilchtröpfchen auf den Matrixteilchen fein verteilt angelagert werden und es zu einer für eine schonende Trocknung wichtigen optimalen feinen Oberflächenverteilung der Milch kommt. Die Milch wird somit auf möglichst geringem Volumen möglichst stark verteilt. Dadurch wird die Milch rasch und unter sanften Bedingungen getrocknet und kann in hoher Konzentration lagerstabil zur Verfügung gestellt werden. Durch die Matrix wird nicht nur eine feine Verteilung der Milch auf einer möglichst großen Oberfläche erreicht, sondern die Matrix bietet auch einen gewissen Schutz vor anderen Stoffen, die die empfindlichen Inhaltsstoffe der Milch, beispielsweise die ungesättigten Fettsäuren, angreifen. Die Milch kann beispielsweise durch Aufsprühen auf die hochdisperse Matrix aufgebracht werden.

Durch die auf diese Weise getrocknete Stutenmilch ist es möglich, die Temperatur- und Sauerstoff-empfindlichen Inhaltsstoffe, insbesondere die Fettsäuren, schonend und ohne Verluste zu konzentrieren und zu trocknen, so dass die hochwertigen Inhaltsstoffe temperaturschonend getrocknet werden. Dadurch wird ein Stutenmilchkonzentrat zur Verfügung gestellt, dass nicht nur eine maximale biologische Wertigkeit aufweist, sondern bei Raumtemperatur lagerstabil ist und sich überraschenderweise besser als die herkömmlichen Präparate zur Behandlung von Hauterkrankungen eignet. Im Vergleich beispielsweise zu einer Behandlung mit sprühgetrockneter Stutenmilch tritt bei der erfindungsgemäßen Verwendung eine Besserung des Krankheitsverlaufes rasch ein und die Heilung ist dauert auch länger an.

Weiters besteht ein vorteil des erfindungsgemäßen Konzentrats in der Möglichkeit, dieses wertvolle Naturprodukt mit biologisch aktiven Zutaten nach Belieben zu kombinieren und auf diese Weise biologisch wirksame und marktfähige Produkte zu entwickeln und zu vertreiben.

Dadurch, dass ein biologisch hochwertiges Konzentrat eingenommen wird, können täglich sehr große Mengen der biologisch aktiven Inhaltsstoffe zugeführt werden, die sehr großen Mengen an nativer Stutenmilch entsprechen. Somit wird die Behandlung für den Patienten einfacher und angenehmer.

Das Präparat kann dabei beispielsweise in Form eines Pulvers, einer Tablette oder einer Kapsel zur Verfügung gestellt werden. Dabei dient das Präparat für die orale Aufnahme.

Die durchschnittliche Teilchengröße der Matrix beträgt beispielsweise etwa maximal 900 nm, vorzugsweise etwa maximal 500 nm, besonders bevorzugt maximal 250 nm, maximal 100 nm, maximal 50 nm, maximal 25 nm, und am meisten bevorzugt maximal 15 nm.

Die Matrix weist vorzugsweise eine durchschnittliche Oberfläche von mindestens 100 m$^2$/g, besonders bevorzugt mindestens 150 m$^2$/g, noch bevorzugter mindestens 200 m$^2$/g, am meisten bevorzugt mindestens 400 m$^2$/g, auf.

Die Stutenmilch kann beispielsweise über Düsen auf die Matrix aufgetragen werden und diese Mischung kann anschließend in einem Mischbehälter, beispielsweise einer Mischschnecke, schonend getrocknet werden, wobei z.B. eine Vakuumtrocknung durchgeführt wird. Der durch Vakuumtrocknung entstandene Dampf kann etwa mit einem Kondensator kondensiert und in einem Wasserbehälter abgeleitet werden.

Der Trocknungsbehälter ist vorzugsweise drehbar und liegend vorgesehen und kann jegliche Größe aufweisen, etwa 500 bis 1000 1. Vorzugsweise ist die Anlage temperatur- bzw. druckgesteuert. Weiters ist es günstig, wenn zusätzliche Parameter wie Mischzeit, Einspritzzeit, Einspritzdruck, Kippwinkel, Vibratoren, Scherkopfzuschaltung usw.,

programmierbar bzw. einstellbar sind. Dadurch wird das Verfahren optimiert, wobei für den Fachmann die optimalen Größen leicht einstellbar sind.

Besonders bevorzugt wird das Präparat zur Behandlung von Neurodermitis bzw. Psoriasis eingesetzt. Diese Hauterkrankungen zählen zu den "trockenen Hauterkrankungen". Es hat sich herausgestellt, dass das auf einer biologisch inerten hochdispersen Matrix getrocknete Stutenmilchkonzentrat sich besonders bevorzugt zur Behandlung von Neurodermitis bzw. Psoriasis eignet. Wie oben bereits beschrieben, ist es aus dem Stand der Technik bekannt, native Stutenmilch für die Behandlung von Neurodermitis bzw. Psoriasis zu verwenden. Es hat sich jedoch gezeigt, dass das schonend getrocknete Stutenmilchkonzentrat gemäß der vorliegenden Anmeldung besonders gut geeignet ist, da dadurch, verglichen mit z.B. sprühgetrockneter Stutenmilch, eine schnellere Heilung bzw. Besserung der Erkrankung eintritt und auch keine zusätzlichen therapeutischen oder diätetischen Maßnahmen im Gegensatz zu einer Behandlung mit auf andere Weise getrockneter Stutenmilch notwendig sind. Im Vergleich zur nativen Stutenmilch ist das schonend getrocknete Konzentrat haltbar und weist die biologisch hochwertigen Inhaltsstoffe in hochkonzentrierter Form auf.

Eine besonders günstige Verwendung wird dadurch zur Verfügung gestellt, dass die Matrix hochdisperses Siliciumdioxid ist. Diese Matrix ist biologisch inert und ist ausreichend hochdispers, um sich für die schonende Trocknung von Stutenmilch bestens zu eignen. Weiters ist Siliciumdioxid zur Herstellung eines oral einzunehmenden Präparates geeignet, da Siliciumdioxid medizinisch völlig unbedenklich ist.

Beispielsweise ist die Matrix aus Aerosil® hergestellt, eine hochdisperse Kieselsäure von über 99,8 % $SiO_2$-Gehalt. Diese Matrix ist aus amorphen kugelförmigen Teilchen aufgebaut, die einen Durchmesser von etwa 10 bis 20 nm besitzen. Bei einem volumen von ca. 15 ml besitzt 1 g Aerosil® eine Oberfläche von 100 bis 400 m$^2$. Diese Matrix eignet sich besonders gut für die erfindungsgemäße Verwendung.

Eine besonders vorteilhafte Verwendung ist weiters dadurch gekennzeichnet, dass das Stutenmilchkonzentrat bei einer Temperatur von 10 bis 50°C, insbesondere 35 bis 40°C, getrocknet wurde. In diesem Temperaturbereich wird eine völlig schonende Trocknung gewährleistet, sodass die biologische Wertigkeit der Stutenmilch erhalten bleibt. Bei dieser Temperatur bleiben alle wichtigen und auch empfindlichen Inhaltsstoffe voll erhalten. Dabei kann der Mischbehälter umfassend die hochdisperse Matrix und die Stutenmilch beispielsweise mittels Steuerung auf einer konstanten Temperatur beheizt werden.

Weiters ist es günstig, wenn das Stutenmilchkonzentrat bei einem Druck von 1 bis 50 mbar, insbesondere 10 bis 30 mbar, getrocknet wurde. In diesem Druckbereich bleiben die biologisch wichtigen Inhaltsstoffe, insbesondere die ungesättigten Fettsäuren, unbeschadet erhalten. In diesem Druckbereich ist weiters eine schonende Trocknung ohne Temperaturschädigung gewährleistet.

Vorzugsweise umfasst das Präparat zusätzlich essentielle Fettsäuren, insbesondere pflanzliche essentielle Fettsäuren. Dies sind insbesondere Linolensäure, Stearidonsäure, Eicosadienolsäure, Linolsäure, Palmitoleinsäure, Vaccensäure, Eicosensäure, Erucasäure, Nervonsäure, Ölsäure. Die Kombination von getrocknetem Stutenmilchkonzentrat mit pflanzlichen essentiellen Fettsäuren hat sich als besonders günstig für die Behandlung von Hauterkrankungen herausgestellt, da dadurch alle notwendigen Stoffe zur Heilung der Erkrankung damit verabreicht werden. Durch die pflanzlichen essentiellen Fettsäuren wird das Stutenmilchkonzentrat optimal ergänzt.

Weiters ist es vorteilhaft, wenn das Präparat weiters zumindest eine Substanz ausgewählt aus der Gruppe bestehend aus Hydrogencarbonat, Kalium, Carbonat, Citrat, Calcium, Magnesium, Vitamin C, Vitamin E, Niacin, Zink, Eisen, beta-Carotin, Pantothensäure, Mangan, Vitamin B6, Vitamin B2, Vitamin B1, Kupfer, Natrium, Biotin, Folsäure, Molybdän, Selen, Xanthan, Fructose, Zitronensäure und Vitamin B12 oder eine Kombination von zumindest zwei dieser Substanzen umfasst.

Wird zumindest eine Substanz oder eine Kombination aus zumindest zwei Substanzen dieser Gruppe dem Stutenmilchkonzentrat zugesetzt, wird dadurch eine ausgesprochen effiziente Kombination zur Verfügung gestellt, da das Stutenmilchkonzentrat optimal ergänzt wird. Dadurch wird ein Präparat zur Verfügung gestellt, dass sich ausgezeichnet zur Behandlung von Hauterkrankungen, insbesondere Neurodermitis und Psoriasis, eignet.

Die vorliegende Erfindung wird nun anhand der nachfolgenden Beispiele näher erläutert, auf die sie jedoch nicht beschränkt sein soll.

**Beispiele**

**Behandlung von Patienten mit Psoriasis und Neurodermitis mit einem Stutenmilchkonzentrat**

Patienten mit Psoriasis und Neurodermitis wurden mit einem Stutenmilchkonzentrat ("Neurodermitis-Cocktail") behandelt, wobei dieses Konzentrat die in Tabelle 1 angeführten Inhaltsstoffe aufweist. Dieses Konzentrat wurde schonend hergestellt, wobei native Stutenmilch auf einer hochdispersen Siliciumdioxid-Matrix fein verteilt aufgebracht und in einem Mischbehälter bei etwa 32°C bei 10 mbar schonend getrocknet. 150 kg Stutenmilch wurden pasteurisiert, anschließend mit 625 g hochdispersem Siliciumdioxid (als inerte Trägermatrix) sowie mit 0,75 g Zitronensäure und 7,50 g D,L-alpha-Tocopherol (als Stabilisatoren) versetzt. Die Mischung wird in einer geschlossenen Evaporations-Anlage bei ca. 32°C und einem Vakuum von 10 mbar unter ständigem Rühren in einem Zeitraum von 24 Stunden zur Trockene aufkonzentriert. Nach dem Trocknungsvorgang wird das Stutenmilch-Trockenkonzentrat mit den in Tabelle 1 angeführten Ölen, Mineralstoffen, Vitaminen und Spurenelementen sowie mit dem Hilfsstoff hochdisperses Siliciumdioxid zu einem Pulver vermischt.

Einnahmeempfehlung: 1 mal täglich, am besten abends vor dem Schlafengehen, eine Portion in Wasser oder Milch mit einem Shaker oder Rührstab einrühren und schluckweise trinken.

Kinder von 1 bis unter 4 Jahren: 1 gestrichenen Esslöffel (ca. 6,67 g) Pulver in 1/8 1 (125 ml) Wasser oder Milch einrühren.

Kinder von 4 bis unter 13 Jahren: 2 gestrichene Esslöffel (ca. 13,3 g) Pulver in ¼ 1 (250 ml) Wasser oder Milch einrühren.

Kinder ab 13 Jahren, Jugendliche und Erwachsene: 3 gestrichene Esslöffel (ca. 20 g) Pulver in ¼ 1 (250 ml) Wasser oder Milch einrühren.

### Tabelle 1

| Nährstoffe pro 1, 2, 3, gestrichene Esslöffel Neurodermitis-Cocktail(EL=Esslöffel; entspricht 6,67 g, 13,3 g, 20 g) | | | |
|---|---|---|---|
| *Dosierung* | *Kinder von 1 bis unter 4 Jahren* | *Kinder von 4 bis unter 13 Jahren* | *Kinder ab 13 a, Jugendliche und Erwachsene* |
| | 1 EL enthält: | 2 EL enthalten: | 3 EL enthalten: |
| Stutenmilch-Trockenkonzentrat | 330 mg | 660 mg | 990 mg |
| omega-3-FS $\alpha$-Linolensäure | 280,1 mg | 560,2 mg | 840,3 mg |
| omega-3-FS Stearidonsäure | 0,3 mg | 0,5 mg | 0,8 mg |
| omega-6-FS Eicosadienolsäure | 0,5 mg | 1,1 mg | 1,6 mg |
| omega-6-FS $\alpha$-Linolensäure | 66,7.mg | 133,3 mg | 200,0 mg |
| omega-6-FS Linolsäure | 448,2 mg | 896,4 mg | 1344,5 mg |
| omega-7-FS Palmitoleinsäure | 0,5 mg | 1,1 mg | 1,6 mg |
| omega-7-FS Vaccensäure | 1,3 mg | 2,7 mg | 4,0 mg |
| omega-9-FS Eicosensäure | 10,7 mg | 21,3 mg | 32,0 mg |
| omega-9-FS Erucasäure | 8,0 mg | 16,0 mg | 24,0 mg |
| omega-9-FS Nervonsäure | 5,3 mg | 10,7 mg | 16,0 mg |
| omega-9-FS Ölsäure | 208,0 mg | 416,0 mg | 624,0 mg |
| Summe essentielle Fettsäuren | 1029,6 mg | 2059,3 mg | 3088,9 mg |
| Hydrogencarbonat | 195,1 mg | 390,3 mg | 585,4 mg |
| Kalium | 166,7 mg | 333,3 mg | 500,0 mg |
| Carbonat | 88,7 mg | 177,3 mg | 266,0 mg |
| Citrat | 67,1 mg | 134,2 mg | 201,3 mg |
| Calcium | 66,7 mg | 133,3 mg | 200,0 mg |
| Magnesium | 66,7 mg | 133,3 mg | 200,0 mg |

(fortgesetzt)

| Nährstoffe pro 1, 2, 3, gestrichene Esslöffel Neurodermitis-Cocktail(EL=Esslöffel; entspricht 6,67 g, 13,3 g, 20 g) | | | |
|---|---|---|---|
| *Dosierung* | *Kinder von 1 bis unter 4 Jahren* | *Kinder von 4 bis unter 13 Jahren* | *Kinder ab 13 a, Jugendliche und Erwachsene* |
| | 1 EL enthält: | 2 EL enthalten: | 3 EL enthalten: |
| Vitamin C | 20,0 mg | 40,0 mg | 60,0 mg |
| Vitamin E | 6,7 mg | 13,3 mg | 20,0 mg |
| Niacin | 5,0 mg | 10,0 mg | 15,0 mg |
| Zink | 4,0 mg | 8,0 mg | 12,0 mg |
| Eisen | 3,3 mg | 6,7 mg | 10,0 mg |
| Beta-Carotin | 2,0 mg | 4,0 mg | 6,0 mg |
| Pantothensäure | 1,7 mg | 3,3 mg | 5,0 mg |
| Mangan | 0,67 mg | 1,3 mg | 2,0 mg |
| Vitamin B6 | 0,53 mg | 1,1 mg | 1,6 mg |
| Vitamin B2 | 0,50 mg | 1,0 mg | 1,5 mg |
| Vitamin B1 | 0,37 mg | 0,73 mg | 1,1 mg |
| Kupfer | 0,17 mg | 0,33 mg | 0,5 mg |
| Natrium | 35 mcg | 71 mcg | 106 mcg |
| Biotin | 33 mcg | 67 mcg | 100 mcg |
| Folsäure | 33 mcg | 67 mcg | 100 mcg |
| Molybdän | 33 mcg | 67 mcg | 100 mcg |
| Selen | 33 mcg | 67 mcg | 100 mcg |
| Vitamin B12 | 0,7 mcg | 1,3 mcg | 2 mcg |

**Untersuchungsparameter:**

**Neurodermitis**

Hauptzielparameter: SCORAD (Severity Scoring of Atopic Dermatitis). Zur qualitativen und quantitativen Einschätzung des Schweregrades des atopischen Ekzems wurde der SCORAD-Index (Severity Scoring of Atopic Dermatitis) verwendet. Dieser erlaubt die standardisierte Beurteilung des Ausprägungsgrades von sechs typischen morphologischen Veränderungen (0-3, max. 18), des Anteils der betroffenen Hautfläche (%) und der subjektiven Einschätzung von Juckreiz und Schlafverlust anhand einer visuellen Analogskala (0-10, max. 20). Einzelne wie auch inhaltliche Gruppen von Parametern oder der Gesamtscore (maximal 103 Punkte) können analysiert werden.

SCORAD-Index orientiert sich an Ausdehnung (a), Intensität (B) und subjektiven Symptomen (C) wie Pruritus und Schlaflosigkeit. Wie man an der SCORAD-Formel A/5 plus 7B/2 plus C leicht erkennt, erhält dabei die Intensität die stärkste Gewichtung. Dabei werden von jedem Schweregrad jeweils fünf verschiedene Hauptsymptome (Erythem, Ödem/Papelbildung, Nässen/Krustenbildung, Exkoriation und Lichenifikation) dargestellt. Die subjektiven Symptome müssen die PatientInnen selbst auf einer visuellen Analogskala eintragen.

Sekundärparameter: Verträglichkeit und Akzeptanz der Prüfsubstanz

Die Berechnungen des SCORAD erfolgten über den SCORAD-Calculator der Universität Nantes (http://scorad.sante.univ-nantes.fr/Compute.html).

**Psoriasis:**

Hauptzielparameter: Beurteilt wurden der Ausbreitungsgrad und Intensität von typischen morphologischen Veränderungen der betroffenen Hautfläche. Als Messinstrument wurde der Psoriasis Area and Severity Index (PASI) eingesetzt. Diese Kennziffer berücksichtigt die Fläche der betroffenen Haut sowie das Ausmaß der Entzündung und übersteigerten Zellteilung. Dazu bestimmt der Untersucher für je einen Herd an Kopf, Körperstamm, Arm und Bein anhand einer von 0 bis 4 reichenden Skala die Rötung, Verdickung und Schuppung. Die Punktzahlen werden mit denen des geschätzten Befalls multipliziert. Daraus ergibt sich nach einem Umrechnungsschlüssel für den prozentualen Anteil der einzelnen Regionen ein PASI zwischen 0 und 96.

Sekundärparamater: Verträglichkeit und Akzeptanz der Prüfsubstanz

**Beispiel 1:**

Teilnehmer Nr. 01
Initialen: JT
Geburtsdatum: 14.09.1991
Geschlecht: männlich
Diagnose: Neurodermitis seit Geburt
Dosierung: Neurodermitis-Cocktail: 2 Esslöffel pro Tag (=13,3 g)

**Tabelle 2 : SCORAD - Patient Nr. 01**

| | Baseline-Visite | 1. Visite nach 1 Monat | 2. Visite nach 2 Monaten | 3. Visite nach 3 Monaten |
|---|---|---|---|---|
| A: Ausmaß (0-102) | 6 | 0 | 0 | 0 |
| B: Intensität (0-18) | 7 | 2 | 2 | 2 |
| C: Subjektive Symptome (0-20) | 5 | 2 | 0 | 0 |
| SCORAD* (0-103) | 31 | 9 | 7 | 7 |
| *SCORAD = A/5+7B/2+C | | | | |

Ausmaß, Intensität, subjektive Symptome und der Summenscore zeigten deutliche Verbesserungen im Therapieverlauf.

Als begleitende Maßnahmen erhielt der Patient während des Supplementationszeitraumes fettende Salben.

Im Studienverlauf berichtete der Patient über keine Nebenwirkungen des Präparates. Geschmacklich wurde das Präparat vom Patienten als "gut" eingestuft.

**Beispiel 2:**

Teilnehmer Nr. 02
Initialen: RA
Geburtsdatum: 18.04.1998
Geschlecht: weiblich
Diagnose: Neurodermitis seit Geburt
Dosierung: Neurodermitis-Cocktail: 1 Esslöffel pro Tag (=6,67 g)

**Tabelle 3 : SCORAD - Patient Nr. 02**

| | Baseline-Visite | 1. Visite nach 1 Monat | 2. Visite nach 2 Monaten | 3. Visite nach 3 Monaten |
|---|---|---|---|---|
| A: Ausmaß (0-102) | 8 | 3 | 1 | 1 |
| B: Intensität (0-18) | 6 | 2 | 2 | 2 |

(fortgesetzt)

|  | Baseline-Visite | 1. Visite nach 1 Monat | 2. Visite nach 2 Monaten | 3. Visite nach 3 Monaten |
|---|---|---|---|---|
| C: Subjektive Symptome (0-20) | 7 | 0 | 0 | 0 |
| SCORAD* (0-103) | 30 | 8 | 7 | 7 |
| *SCORAD = A/5+7B/2+C | | | | |

Ausmaß, Intensität, subjektive Symptome und der Summenscore zeigten deutliche Verbesserungen im Therapieverlauf.

Als begleitende Maßnahmen erhielt die Patientin während des Supplementationszeitraumes fettende Salben und Ölbäder.

Im Studienverlauf berichtete die Patientin über keine Nebenwirkungen des Präparates. Geschmacklich wurde das Präparat von der Patientin als "sehr gut" eingestuft.

**Beispiel 3:**

Teilnehmer Nr. 04
Initialen: ZM
Geburtsdatum: 17.02.1968
Geschlecht: weiblich
Diagnose: Neurodermitis seit Geburt
Dosierung: Neurodermitis-Cocktail: 3 Esslöffel pro Tag (=20 g)

## Tabelle 4 : SCORAD - Patient Nr. 04

|  | Baseline-Visite | 1. Visite nach 1 Monat | 2. Visite nach 2 Monaten | 3. Visite nach 3 Monaten |
|---|---|---|---|---|
| A: Ausmaß (0-102) | 12 | 6 | - | 1 |
| B: Intensität (0-18) | 7 | 3 | - | 2 |
| c: Subjektive Symptome (0-20) | 8 | 3 | - | 0 |
| SCORAD* (0-103) | 35 | 15 | - | 7 |
| *SCORAD = A/5+7B/2+C | | | | |

Ausmaß, Intensität, subjektive Symptome und der Summenscore zeigten deutliche Verbesserungen im Therapieverlauf.

Als begleitende Maßnahmen erhielt die Patientin während des Supplementationszeitraumes fettende Salben und Ölbäder.

Im Studienverlauf berichtete die Patientin über keine Nebenwirkungen des Präparates. Geschmacklich wurde das Präparat von der Patientin als "sehr gut" eingestuft.

Die drei dokumentierten Neurodermitis-Fälle zeigten initial eine mittelschwere Form (SCORAD 30 bis 35) der atopischen Dermatitis. Die Ergebnisse der Therapie mit Neurodermitis-Cocktail zeigten bei den Studienteilnehmern/ Innen eine deutliche und anhaltende Besserung des Hautbefundes, welcher bis Studienende erhalten blieb (SCORAD 7 - leichte Form der Neurodermitis - nach 12 Wochen Supplementation).

**Psoriasis**

Zur qualitativen und quantitativen Einschätzung des Schweregrades der Schuppenflechte wurde der PASI (Psoriasis Area Severity Index) verwendet.

PASI für die einzelnen Hautabschnitte (http://members.aol.com/psorsite/docs/pasi.html):

Hautabschnitt Beine:

Ersatzseite

- $(Juckreiz_{Beine} + Rötung_{Beine} + Schuppen_{Beine} + Hautdicke_{Beine})$ $\times Ausbreitung_{Beine} \times 0,4 = Gesamtsumme_{Beine}$

Hautabschnitt Rumpf:
- $(Juckreiz_{Rumpf} + Rötung_{Rumpf} + Schuppen_{Rumpf} + Hautdicke_{Rumpf})$ $\times Ausbreitung_{Rumpf} \times 0,3 = Gesamtsumme_{Rumpf}$

Hautabschnitt Arme:
- $(Juckreiz_{Arme} + Rötung_{Arme} + Schuppen_{Arme} + Hautdicke_{Arme})$ $\times Ausbreitung_{Arme} \times 0,2 = Gesamtsumme_{Arme}$

Hautabschnitt Kopf:
- $(Juckreiz_{Kopf} + Rötung_{Kopf} + Schuppen_{Kopf} + Hautdicke_{Kopf})$ $\times Ausbreitung_{Kopf} \times 0,1 = Gesamtsumme_{Kopf}$

$$PASI\ gesamt = Gesamtsumme_{Beine} + Gesamtsumme_{Rumpf} + Gesamtsumme_{Arme} + Gesamtsumme_{Kopf}$$

**Beispiel 4:**

Teilnehmer Nr. 01
Initialen: SG
Geburtsdatum: 12.03.1943
Geschlecht: weiblich
Diagnose: Psoriasis seit 5 Jahren
Dosierung: Neurodermitis-Cocktail: 3 Esslöffel pro Tag (=20 g)

**Tabelle 5: PASI-Psoriasis Area and Severity Index-Patient Nr. 01**

| PASI | Baseline-Visite | 1. Visite nach 6 Wochen | 2. Visite nach 14 Wochen ab Baseline | 3. Visite nach 16 Wochen ab Baseline |
|---|---|---|---|---|
| Hautabschnitt Beine | 6,4 | 0,8 | 0,8 | 1,2 |

(fortgesetzt)

| PASI | Baseline-Visite | 1. Visite nach 6 Wochen | 2. Visite nach 14 Wochen ab Baseline | 3. Visite nach 16 Wochen ab Baseline |
|---|---|---|---|---|
| Hautabschnitt Rumpf | 0 | 0 | 0 | 0 |
| Hautabschnitt Arme | 2,4 | 0,2 | 0 | 0,2 |
| Hautabschnitt Kopf | 0 | 0 | 0 | 0 |
| PASI gesamt | 8 , 8 | 1 , 0 | 0,8 | 1 , 4 |

Deutliche Verbesserungen des PASI konnten in den einzelnen Hautabschnitten sowie im PASI gesamt erzielt werden.

Als begleitende Maßnahmen erhielt die Patientin während des gesamten Supplementationszeitraumes topische Kortikosteroide, Keratolytika und wirkstofffreie Cremes und Salben.

Im Studienverlauf berichtete die Patientin über keine Nebenwirkungen des Präparates. Geschmacklich wurde das Präparat von der Patientin als "gut" eingestuft.

**Beispiel 5:**

Teilnehmer Nr. 02
Initialen: WA
Geburtsdatum: 03.05.1959
Geschlecht: weiblich
Diagnose: Psoriasis seit 3 Jahren
Dosierung: Neurodermitis-Cocktail: 3 Esslöffel pro Tag (=20 g)

**Tabelle 6: PASI-Psoriasis Area and Severity Index-Patient Nr. 02**

| PASI | Baseline-Visite | 1. Visite nach 4 Wochen | 2. Visite nach 9 Wochen ab Baseline | 3. Visite nach 17 Wochen ab Baseline |
|---|---|---|---|---|
| Hautabschnitt Beine | 2,0 | 0,4 | 0,4 | 0,4 |
| Hautabschnitt Rumpf | 0 | 0 | 0 | 0 |
| Hautabschnitt Arme | 1,0 | 0,4 | 0,2 | 0,2 |
| Hautabschnitt Kopf | 0,6 | 0,2 | 0 | 0 |
| PASI gesamt | 3,6 | 1,0 | 0,6 | 0,6 |

Eine Verbesserung des PASI konnte in den einzelnen Hautabschnitten sowie im PASI gesamt erzielt werden. Als begleitende Maßnahmen erhielt die Patientin von der Baseline-Visite bis zur 1. Visite nach 4 Wochen wirkstofffreie Cremes und Salben.

Im Studienverlauf berichtete die Patientin über keine Nebenwirkungen des Präparates. Geschmacklich wurde das Präparat von der Studienteilnehmerin als "sehr gut" eingestuft.

**Beispiel 6:**

Teilnehmer Nr. 04
Initialen: GA
Geburtsdatum: 24.06.1946
Geschlecht: weiblich
Diagnose: Psoriasis seit 3 Jahren
Dosierung: Neurodermitis-Cocktail: 3 Esslöffel pro Tag (=20 g)

**Tabelle 7: PASI-Psoriasis Area and Severity Index-Patient Nr. 04**

| PASI | Baseline-Visite | 1. Visite nach 3 Wochen | 2. Visite nach 7 ½ Wochen ab Baseline | 3. Visite nach 13 Wochen ab Baseline |
|---|---|---|---|---|
| Hautabschnitt Beine | 0 | 0 | 0 | 0 |
| Hautabschnitt Rumpf | 1,5 | 0,9 | 0,3 | 0 |
| Hautabschnitt Arme | 0 | 0 | 0 | 0 |
| Hautabschnitt Kopf | 0,5 | 0,2 | 0,1 | 0,4 |
| PASI gesamt | 2,0 | 1,1 | 0,4 | 0,4 |

Eine Verbesserung des PASI konnte erzielt werden. Im Abschnitt Rumpf konnte durch die Neurodermitis-Cocktail-Gabe eine komplette Remission erzielt werden.
Als begleitende Maßnahmen erhielt die Patientin während des gesamten Supplementationszeitraumes topische Kortikosteroide.
Im Studienverlauf berichtete die Patientin über keine Nebenwirkungen des Präparates. Geschmacklich wurde das Präparat von der Studienteilnehmerin als "mittel" eingestuft.

Die drei dokumentierten Psoriasis-Fälle hatten initial einen PA-SI von 8,8 bzw. 3,6 bzw. 2,0. Eine deutliche und anhaltende Besserung des Hautbefundes, welcher bis Studienende erhalten blieb (PASI 1,4 bzw. 0,6 bzw. 0,4) konnte unter Therapie mit Neurodermitis-Cocktail gezeigt werden.

Die überraschend guten Ergebnisse der Beobachtungsstudie bestätigen den ernährungsmedizinischen Therapieansatz, temperaturschonend konzentrierte Stutenmilch als Basis für die Therapie von Hauterkrankungen einzusetzen. Trotz geringer Fallzahl kann die Erfolgsrate von 100 % im Vergleich zu herkömmlichen Therapie-Ansätzen als überdurchschnittlich erfolgreich bezeichnet werden. Obwohl der absolute Gehalt an gamma-Linolensäure in der eingesetzten Nährstoff-Mischung gering war, konnte - vermutlich durch Aktivierung des Enzyms delta-6-Desaturase - die Metabolisierung alimentärer Vorstufen wie zum Beispiel der omega-6-Fettsäure "Linolsäure" stimuliert werden.

**Patentansprüche**

1. Verwendung eines auf einer biologisch inerten, hochdispersen Matrix getrockneten Stutenmilchkonzentrats zur Herstellung eines Präparats zur oralen Aufnahme zur Behandlung von Hauterkrankungen, insbesondere trockenen Hauterkrankungen.

2. Verwendung nach Anspruch 1, **dadurch gekennzeichnet, dass** das Präparat zur Behandlung von Neurodermitis einzusetzen ist.

3. Verwendung nach Anspruch 1, **dadurch gekennzeichnet, dass** das Präparat zur Behandlung von Psoriasis einzusetzen ist.

4. Verwendung nach einem der Ansprüche 1 bis 3, **dadurch gekennzeichnet, dass** die Matrix hochdisperses Siliciumdioxid ist.

5. Verwendung nach einem der Ansprüche 1 bis 4, **dadurch gekennzeichnet, dass** das Stutenmilchkonzentrat durch Trocknung bei einer Temperatur von 10-50°C, insbesondere 35-90°C, herstellbar ist.

6. Verwendung nach einem der Ansprüche 1 bis 5, **dadurch gekennzeichnet, dass** das Stutenmilchkonzentrat durch Trocknung bei einem Druck von 1-50 mbar, insbesondere 10-30 mbar, herstellbar ist.

7. Verwendung nach einem der Ansprüche 1 bis 6, **dadurch gekennzeichnet, dass** das Präparat zusätzliche essentielle Fettsäuren, insbesondere pflanzliche essentielle Fettsäuren, umfasst.

8. Verwendung nach einem der Ansprüche 1 bis 7, **dadurch gekennzeichnet, dass** das Präparat weiters zumindest eine Substanz ausgewählt aus der Gruppe bestehend aus Hydrogencarbonat, Kalium, Carbonat, Citrat, Calcium, Magnesium, Vitamin C, Vitamin E, Niacin, Zink, Eisen, Beta Carotin, Pantothensäure, Mangan, Vitamin B6, Vitamin B2, Vitamin B1, Kupfer, Natrium, Biotin, Folsäure, Molybdän, Selen, Xanthan, Fructose, Zitronensäure und Vitamin B12 oder eine Kombination von zumindest zwei dieser Substanzen umfasst.

**Claims**

1. The use of a mare milk concentrate dried on a biologically inert, highly disperse matrix for the production of a preparation for oral intake, for the treatment of skin diseases and, in particular, dry skin diseases.

2. The use according to claim 1, **characterized in that** the preparation is to be used for the treatment of neurodermatitis.

3. The use according to claim 1, **characterized in that** the preparation is to be used for the treatment of psoriasis.

4. The use according to any one of claims 1 to 3, **characterized in that** the matrix is a highly disperse silicon dioxide.

5. The use according to any one of claims 1 to 4, **characterized in that** the mare milk concentrate is producible by drying at a temperature of from 10 to 50°C and, in particular 35 to 40°C.

6. The use according to any one of claims 1 to 5, **characterized in that** the mare milk concentrate is producible by drying at a pressure of from 1 to 50 mbar and, in particular, 10 to 30 mbar.

7. The use according to any one of claims 1 to 6, **characterized in that** the preparation additionally comprises essential fatty acids and, in particular, vegetable essential fatty acids.

8. The use according to any one of claims 1 to 7, **characterized in that** the preparation additionally comprises at least one substance selected from the group consisting of hydrogen carbonate, potassium, carbonate, citrate, calcium, magnesium, vitamin C, vitamin E, niacin, zinc, iron, beta-carotene, pantothenic acid, manganese, vitamin B6, vitamin B2, vitamin B1, copper, sodium, biotin, folic acid, molybdenum, selenium, xanthan, fructose, citric acid and vitamin B12 or a combination of at least two of these substances.

**Revendications**

1. Utilisation d'un concentré de lait de jument, séché sur une matrice hautement dispersée et biologiquement inerte, pour fabriquer une préparation destinée à une ingestion orale, pour le traitement de maladies de la peau, en particulier de maladies de peau sèche.

2. Utilisation selon la revendication 1, **caractérisée en ce que** la préparation est à utiliser pour le traitement d'une névrodermite.

3. Utilisation selon la revendication 1, **caractérisée en ce que** la préparation est à utiliser pour le traitement du psoriasis.

4. Utilisation selon l'une des revendications 1 à 3, **caractérisée en ce que** la matrice est du dioxyde de silicium hautement dispersé.

5. Utilisation selon l'une des revendications 1 à 4, **caractérisée en ce que** le concentré de lait de jument peut être préparé par séchage à une température de 10 à 50 °C, en particulier de 35 à 40 °C.

6. Utilisation selon l'une des revendications 1 à 5, **caractérisée en ce que** le concentré de lait de jument peut être préparé par séchage sous une pression de 1 à 50 mbar, en particulier de 10 à 30 mbar.

7. Utilisation selon l'une des revendications 1 à 6, **caractérisée en ce que** la préparation contient des acides gras essentiels additionnels, en particulier des acides gras essentiels végétaux.

8. Utilisation selon l'une des revendications 1 à 7, **caractérisée en ce que** la préparation comprend en outre au moins une substance choisie dans l'ensemble constitué d'hydrogénocarbonates, de potassium, de carbonates, de citrates, de calcium, de magnésium, de vitamine C, de vitamine E, de niacine, de zinc, de fer, de bêta-carotène, d'acide pantothénique, de manganèse, de vitamine B6, de vitamine B2, de vitamine B1, de cuivre, de sodium, de biotine, d'acide folique, de molybdène, de sélénium, de xanthane, de fructose, d'acide citrique et de vitamine B12, ou une combinaison d'au moins deux de ces substances.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- AT 393961

**In der Beschreibung aufgeführte Nicht-Patentliteratur**

- **ALEXANDER BÜHLBÄCKER.** Zur Verwendbarkeit von Stutenmilch, Kumyß und Eselmilch als Diätetika und Heilmittel unter besonderer Berücksichtigung der Bedürfnisse des Säuglings und des Frühgeborenen. Verlag Dr. Markus Hänsel-Hohenhausen, 1996, 367-376